# FASCICULE DE BREVET EUROPEEN

(11) **EP 0 646 109 B1**
(45) Date de publication et mention de la délivrance du brevet: **03.09.1997**
(21) Numéro de dépôt: 93913127.2
(22) Date de dépôt: 15.06.1993
(51) Int. Cl.: C07C 215/16, A61K 7/13

(54) **NOUVELLES 2-NITRO P-PHENYLENEDIAMINES HYDROXYETHYLEES ET LEUR UTILISATION EN TEINTURE DES FIBRES KERATINIQUES**
HYDROXYLETHYLIERTE 2-NITRO-P-PHENYLDIAMINE UND IHRE VERWENDUNG ALS FÄRBEMITTEL VON KERATINISCHEN FASERN
NOVEL HYDROXYETHYLATED 2-NITRO-P-PHENYLENEDIAMINES AND USE THEREOF FOR DYEING KERATIN FIBRES

(30) Priorité: 19.06.1992 FR 9207516
(43) Date de publication de la demande: 05.04.1995
(73) Titulaire: L'OREAL, 75008 Paris (FR)
(72) Inventeur: LAGRANGE, Alain, F-78400 Chatou (FR); JUNINO, Alex, F-93190 Livry-Gargan (FR); GENET, Alain, F-93600 Aulnay-sous-Bois (FR); COTTERET, Jean, F-78480 Verneuil-sur-Seine (FR)
(74) Mandataire: Casalonga, Axel
(86) Numéro de dépôt international: FR9300571
(87) Numéro de publication internationale: WO9400415

(56) Documents cités:
- EP-A- 0 184 061
- FR-A- 1 454 314

## Description

La présente invention concerne de nouveaux colorants nitrés benzéniques du type 2-nitro p-phénylènediamine, destinés à la teinture des fibres kératiniques, et en particulier des cheveux humains.

Dans le domaine de la coloration capillaire, l'utilisation des colorants directs est très répandue car ils présentent certains avantages par rapport aux précurseurs de colorants d'oxydation et, notamment, une diminution des risques potentiels d'allergie et l'absence de sensibilisation du cheveu due au processus oxydatif.

Parmi les colorants directs les plus utilisés figurent les dérivés nitrés benzéniques qui, d'une part, présentent une forte affinité pour le cheveu et qui, d'autre part, grâce à la variété des substituants possibles, permettent de couvrir une large gamme de nuances allant du jaune au bleu en passant par le rouge.

Parmi les colorants nitrés bleu ou pourpre bleu utilisés, on peut citer le 1-β-hydroxyéthylamino-4-N,N-bis(β-hydroxyéthyl)amino-2-nitrobenzène décrit dans le brevet français 1 101 904, le 1-β-hydroxyéthylamino-4-(N-méthyl, N-β-hydroxyéthyl)amino-2-nitrobenzène décrit dans le brevet canadien 900 490, ainsi que le 1-β-hydroxyéthylamino-4-(N-éthyl, N-β-hydroxyéthyl)amino-2-nitro-benzène décrit dans le brevet EP-0 184 061.

Toutefois, la formulation de ces colorants nitrés pose des problèmes du fait de leur résistance au lavage, qui n'est pas satisfaisante.

La demanderesse a donc recherché d'autres colorants nitrés benzéniques ayant des nuances s'échelonnant du bleu au pourpre, présentant une bonne solubilité dans l'eau, dans les mélanges eau/alcool et plus généralement dans les supports de teinture usuels et qui conduisent, sur cheveux, à des teintures stables au lavage et aussi à la lumière, aux intempéries, et à la transpiration.

C'est à la suite de ces recherches que la demanderesse a découvert de nouvelles 2-nitro p-phénylènediamines ayant pour formule : 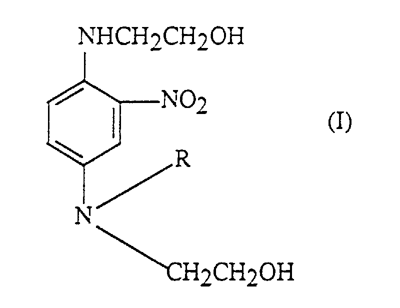 dans laquelle R représente un radical alkyle linéaire ou ramifié comportant 3 ou 4 atomes de carbone.

Parmi les radicaux alkyle R, on peut citer les radicaux n-propyle, isopropyle, n-butyle, isobutyle et tert.-butyle, les radicaux préférés étant n-propyle et isobutyle.

Les composés de formule (I) peuvent être utilisés sous forme de base libre ou salifiée par des acides tels que l'acide chlorhydrique, l'acide bromhydrique, l'acide sulfurique, etc. Ils peuvent donc se trouver sous forme de chlorhydrate, bromhydrate, sulfate, etc.

La présente invention a donc pour objet les nouveaux composés de formule (I) ainsi que leurs sels cosmétiquement acceptables.

Les composés de formule (I) sont préparés par un procédé d'alkylation classique consistant à faire réagir en milieu aqueux et à température comprise entre la température ambiante et la température de reflux du milieu, le 1,4-di-(β-hydroxyéthylamino)-2-nitrobenzène avec un halogéno alcane tel qu'un bromo-, iodo- ou chloro-alcane, en présence de carbonate de calcium, puis à purifier éventuellement le composé obtenu.

L'invention a également pour objet une composition tinctoriale pour la coloration directe des fibres kératiniques et en particulier des cheveux humains, contenant dans un véhicule aqueux, alcoolique ou hydroalcoolique, au moins un composé de formule (I) ou l'un de ses sels cosmétiquement acceptables.

La demanderesse a constaté que l'association d'un colorant nitré benzénique bleu à pourpre de formule (I), dans des compositions de teinture directe, avec des colorants jaune ou jaune vert et éventuellement avec des colorants orangés ou rouges, conduit à l'obtention de nuances naturelles solides au lavage, à la lumière, aux intempéries et à la transpiration.

Selon un mode de réalisation préférentiel, la composition tinctoriale selon l'invention contient donc un composé de formule (I) ou l'un de ses sels cosmétiquement acceptables en association avec un ou plusieurs colorants nitrés benzéniques jaune ou jaune vert donnant sur cheveux gris à 90% de blancs une nuance ou "hue" comprise entre 2,5 Y et 2,5 GY sur le cercle de Munsell (voir publication de Official Digest, Avril 1975, page 375, figure 2).

Selon un mode de réalisation plus particulièrement préféré de la présente invention, le composé de formule (I) est associé aux colorants jaune ou jaune vert choisis parmi les composés suivants :
**Colorants jaune ou jaune vert**
- 1-β-hydroxyéthyloxy-3-méthylamino-4-nitrobenzène,
- 1-(méthylamino)-2-nitro-5- (β,γ-dihydroxypropyl)oxybenzène,
- 1-(β-hydroxyéthylamino)-2-méthoxy-4-nitrobenzène,
- 1-(β-aminoéthylamino)-2-nitro-5-méthoxy-benzène,
- 1,3-di(β-hydroxyéthylamino)-4-nitro-6-chlorobenzène,
- 1-amino-2-nitro-6-méthyl-benzène,
- 1-(β-hydroxyéthylamino)-2-hydroxy-4-nitrobenzène,
- N-(β-hydroxyéthyl)-2-nitro-4-trifluorométhylaniline,
- acide 4-β-hydroxyéthylamino-3-nitro-benzènesulfonique,
- acide 4-éthylarnino-3-nitrobenzoïque,
- 4-(β-hydroxyéthyl)amino-3-nitro-chlorobenzène,
- 4-(β-hydroxyéthyl)amino-3-nitro-méthylbenzène,
- 4-(β,γ-dihydroxypropyl)amino-3-nitro-trifluorométhylbenzène
- 1-β-uréidoéthylamino-4-nitrobenzène,
- O,N-bis(β-hydroxyéthyl)-2-amino-5-nitrophénol,
- 1,3-diamino-4-nitrobenzène,
- 1-hydroxy-2-amino-5-nitrobenzène,
- 1-amino-2-[tris(hydroxyméthyl)méthyl]amino-5-nitro-benzène,
- 1-(β-hydroxyéthyl)amino-2-nitrobenzène,
- 4-(β-hydroxyéthylamino)-3-nitrobenzamide.

Le composé de formule (I) peut également être associé aux colorants rouges ou orangés suivants:
**Colorants rouges**
- 1-hydroxy-3-nitro-4-(γ-hydroxypropylamino)benzène,
- N-(β-hydroxyéthyl)amino-3-nitro-4-aminobenzène,
- 1-amino-3-méthyl-4-(β-hydroxyéthyl)amino-6-nitrobenzène,
- 1-hydroxy-3-nitro-4-N-β-hydroxyéthyl aminobenzène,
- 1,4-diamino-2-nitrobenzène,
- 1-amino-2-nitro-4-méthylaminobenzène,
- N-(β-hydroxyéthyl)-2-nitro-paraphénylènediamine,
- 1-amino-2-nitro-4-(β-hydroxyéthylamino)5-chlorobenzène,
- 2-nitro-4-amino-diphénylamine
- 1-amino-3-nitro-6-hydroxybenzène.
**Colorants orangés**
- 1-(β-aminoéthyl)amino-2-nitro-4-(β-hydroxyéthyl)oxybenzène,
- 1-(β,γ-dihydroxypropyl)oxy-3-nitro-4-(β-hydroxyéthyl)aminobenzène,
- 1-hydroxy-3-nitro-4-aminobenzène,
- 1-hydroxy-2-amino-4,6-dinitrobenzène,
- 1-méthoxy-3-nitro-4-(β-hydroxyéthylamino)benzène,
- 2-nitro-4'-hydroxydiphénylamine,
- 1-amino-2-nitro-4-hydroxy-5-méthylbenzène.

La composition tinctoriale selon l'invention contient 0,01 à 10% en poids, et de préférence 0,1 à 5% en poids, exprimé en base libre, de colorant nitré benzénique de formule (I).

La concentration totale en colorants jaune ou jaune vert peut être comprise entre 0,05 et 3% en poids, sur la base du poids total de la composition tinctoriale.

On peut bien entendu ajouter à la composition tinctoriale selon l'invention d'autres colorants directs tels que des colorants azoïques, des colorants anthraquinoniques, des colorants dérivés du triarylméthane ou des colorants basiques parmi lesquels on peut citer plus particulièrement les colorants connus sous les dénominations "Basic Brown 16", "Basic Yellow 57", "Basic Red 76" et "Basic Blue 99" dans le COLOR INDEX, 3e édition.

La proportion des colorants d'addition, nitrés benzéniques rouges ou orangés ou autres colorants directs, peut varier entre 0,05 et 10% en poids de la composition.

La composition tinctoriale selon l'invention peut comprendre, comme véhicule approprié, l'eau et/ou des solvants organiques acceptables sur le plan cosmétique et plus particulièrement des alcools tels que l'alcool éthylique, l'alcool isopropylique, l'alcool benzylique et l'alcool phényléthylique ou des glycols ou éthers de glycols tels que, par exemple, l'éthylèneglycol et ses éthers monométhylique, monoéthylique et monobutylique, le propylèneglycol, le butylèneglycol, le dipropylèneglycol ainsi que les alkyléthers de diéthylèneglycol, comme par exemple le monoéthyléther ou le monobutyléther du diéthylèneglycol, dans des concentrations comprises entre 0,5 et 20% et, de préférence, entre 2 et 10% par rapport au poids total de la composition.

On peut également ajouter à la composition selon l'invention des amides gras tels que les mono- et diéthanolamides des acides dérivés du coprah, de l'acide laurique ou de l'acide oléique, à des concentrations comprises entre 0,05 et 10% en poids.

On peut aussi ajouter à la composition selon l'invention des agents tensio-actifs anioniques, cationiques, non-ioniques, amphotères ou leurs mélanges. De préférence, les tensio-actifs sont présents dans la composition selon l'invention en une proportion comprise entre 0,1 et 50% en poids et avantageusement, entre 1 et 20% en poids par rapport au poids total de la composition.

Parmi les agents tensio-actifs, on peut citer plus particulièrement les agents tensio-actifs anioniques utilisés seuls ou en mélange tels que, notamment, les sels alcalins, les sels de magnésium, les sels d'ammonium, les sels d'amine ou les sels d'alcanolamine des composés suivants :
- alkylsulfates, alkyléthersulfates, alkylamidesulfates éthoxylés ou non, alkylsulfonates, alkylamide sulfonates, alphaoléfinesulfonates;
- alkylsulfoacétates, alkylphosphates;
- acides gras tels que les acides laurique, myristique, oléique, ricinoléïque, palmitique, stéarique, les acides d'huile de coprah ou d'huile de coprah hydrogénée, les acides carboxyliques d'éthers polyglycoliques,

les radicaux alkyle de ces composés ayant une chaîne linéaire de 12 à 18 atomes de carbone.

A titre d'agents tensio-actifs cationiques, on peut citer plus particulièrement les sels d'amines grasses, les sels d'ammonium quaternaire tels que les chlorures et bromures d'alkyldiméthylbenzylammonium, d'alkyltriméthylammonium, d'alkyldiméthylhydroxyéthylammonium, de diméthyldialkylammonium, les sels d'alkylpyridinium, les dérivés d'imidazoline. Les groupements alkyle des dérivés d'ammonium quaternaire précités sont des groupements à chaîne longue ayant, de préférence, entre 12 et 18 atomes de carbone.

Parmi ces composés à caractère cationique, on peut également citer les oxydes d'amines.

Parmi les agents tensio-actifs amphotères qui peuvent être utilisés, on peut citer en particulier les alkylamino (mono- et di) propionates, les bétaines telles que les alkyl-bétaïnes, les N-alkyl-sulfobétaïnes, les N-alkylaminobétaïnes, le radical alkyle ayant entre 8 et 22 atomes de carbone, les cycloimidiniums tels que les alkylimidazolines.

Parmi les tensio-actifs non ioniques qui peuvent éventuellement être utilisés dans les compositions conformes à l'invention, on peut mentionner les alcools, α-diols, alkylphénols et amides, polyglycérolés, ces composés comportant une chaîne grasse en C₈-C₁₈;
les alcools, alkylphénols et acides gras, polyéthoxylés, ces composés comportant une chaîne grasse en C₈ à C₁₈;
les condensats d'oxyde d'éthylène et de propylène sur des alcools gras; les amides gras polyéthoxylés, contenant au moins 5 moles d'oxyde d'éthylène;
les amines grasses polyéthoxylées.

Les produits épaississants, que l'on peut ajouter dans la composition selon l'invention, peuvent avantageusement être pris dans le groupe formé par l'alginate de sodium, la gomme arabique, la gomme de guar, la gomme de xanthane, la gomme de caroube, les dérivés de la cellulose tels que la méthylcellulose, l'hydroxyéthylcellulose, l'hydroxypropylméthylcellulose, le sel de sodium de la carboxyméthylcellulose et les polymères d'acide acrylique.

On peut également utiliser des agents épaississants minéraux tels que la bentonite. Ces épaississants sont utilisés seuls ou en mélange, et, de préférence, sont présents en une proportion comprise entre 0,2 et 5% en poids par rapport au poids total de la composition et, avantageusement, entre 0,5 et 3% en poids.

La composition tinctoriale selon l'invention peut être formulée à pH acide, neutre ou alcalin, le pH pouvant varier de 4 à 10,5 et, de préférence, de 5 à 10. Parmi les agents d'alcalinisation qui peuvent être utilisés, on peut mentionner les alcanolamines, les hydroxydes et les carbonates alcalins ou d'ammonium. Parmi les agents d'acidification qui peuvent être utilisés, on peut mentionner l'acide lactique, l'acide acétique, l'acide tartrique, l'acide phosphorique, l'acide chlorhydrique et l'acide citrique.

La composition tinctoriale selon l'invention peut contenir en outre divers adjuvants usuels tels que des agents anti-oxydants, des parfums, des agents séquestrants, des produits filmogènes, des agents de traitement du cheveu, des agents dispersants, des agents de conditionnement du cheveu, des agents conservateurs, des agents opacifiants, ainsi que tout autre adjuvant utilisé habituellement en cosmétique.

La composition tinctoriale selon l'invention peut se présenter sous les diverses formes usuelles pour la teinture des cheveux, telles que des liquides épaissis ou gélifiés, des crèmes, des mousses en aérosols ou sous toutes autres formes appropriées pour réaliser une teinture de fibres kératiniques.

La présente invention a également pour objet un procédé de teinture des fibres kératiniques, et notamment des cheveux humains, consistant à laisser agir la composition tinctoriale ci-dessus définie sur les fibres kératiniques séches ou humides. On peut utiliser la composition selon l'invention en tant que lotion non rincée, c'est-à-dire qu'on applique la composition selon l'invention sur les fibres kératiniques, puis on sèche sans rinçage intermédiaire. Dans les autres modes d'application, on applique la composition tinctoriale selon l'invention sur les fibres kératiniques pendant un temps de pose variant entre 3 et 60 minutes, de préférence entre 5 et 45 minutes, on rince, éventuellement on lave et on rince à nouveau, puis on sèche.

Pour mieux faire comprendre l'objet de l'invention, on va en décrire maintenant, à titre d'exemples purement illustratifs et non limitatifs,plusieurs modes de mise en oeuvre.

### EXEMPLES DE PREPARATION

### EXEMPLE 1

**Préparation du chlorhydrate de 1-(β-hydroxyéthyl)amino-4-(N-β-hydroxyéthyl, N-n-propyl)amino-2-nitrobenzène, dénommé selon la nomenclature IUPAC 2-{4-[(2-hydroxyéthyl)propylamino]-2-nitrophénylamino}-éthanol.**

On chauffe au bain d'eau chaude (85°C) la suspension de 48,2 g (0,2 mole) de 1,4-di-(β-hydroxyéthylamino)-2-nitrobenzène et de 30 g de carbonate de calcium dans 80 ml d'eau. En une heure, on ajoute goutte à goutte 54,7 ml (0,6 mole) de 1-bromopropane et on continue le chauffage 7 heures en suivant la réaction en chromatographie en couche mince (gel de silice; éluant: acétate d'éthyle).

On filtre à chaud et on refroidit: l'huile qui précipite est extraite à l'acétate d'éthyle.

La phase acétate d'éthyle est séchée sur sulfate de sodium, filtrée et évaporée à sec sous pression réduite.

L'huile obtenue est purifiée par passage sur colonne moyenne pression (gel de silice; gradient d'acétate d'éthyle et d'heptane).

Après évaporation à sec du solvant, la base libre est dissoute dans 300 ml d'éthanol absolu et on ajoute 20 ml d'une solution d'acide chlorhydrique environ 7N dans l'éthanol absolu.

Le chlorhydrate du composé attendu précipite en cristaux jaunes qui sont essorés, lavés à l'éther éthylique et séchés sur potasse sous vide.

On obtient 36,0 g de chlorhydrate qui fond avec décomposition à 168-170°C et dont l'analyse élémentaire calculée pour C₁₃H₂₂N₃O₄Cl est :

| | C% | H% | N% | O% | Cl% |
|---|---|---|---|---|---|
| Calculé : | 48,83 | 6,93 | 13,14 | 20,01 | 11,09 |
| Trouvé : | 49,08 | 6,95 | 12,99 | 20,10 | 10,98 |

### EXEMPLE 2

**Préparation du chlorhydrate de 1-(β-hydroxyéthyl)amino-4-(N-β-hydroxyéthyl, N-n-butyl)amino-2-nitrobenzène, dénommé selon la nomenclature IUPAC 2-{4-[butyl-(2-hydroxyéthyl)-amino]-2-nitrophénylamino}-éthanol.**

Ce composé est préparé et purifié selon le mode opératoire décrit pour l'exemple précédent.

A partir de 48,2 g (0,2 mole) de 1,4-di (β-hydroxyéthylamino)-2-nitrobenzène et de 1-iodo-butane, on obtient des cristaux jaunes de chlorhydrate (35,0 g) qui fondent avec décomposition à 160-162°C et dont l'analyse élémentaire calculée pour C₁₄H₂₄N₃O₄Cl est:

| | C% | H% | N% | O% | Cl% |
|---|---|---|---|---|---|
| Calculé : | 50,37 | 7,25 | 12,59 | 19,17 | 10,62 |
| Trouvé : | 50,45 | 7,34 | 12,45 | 19,06 | 10,56 |

### EXEMPLE 3

**Préparation du chlorhydrate de 1-(β-hydroxyéthyl)amino-4-(N-β-hydroxyéthyl, N-isobutyl)amino-2-nitrobenzène, dénommé selon la nomenclature IUPAC 2-{4-[(2-hydroxyéthyl)-isobutylamino]-2-nitro-phénylamino}-éthanol.**

Ce composé est préparé et purifié selon le mode opératoire décrit pour l'exemple 1.

A partir de 48,2 g (0,2 mole) de 1,4-di (β-hydroxyéthylamino)-2-nitrobenzène et de 1-iodo-2-méthyl-propane, on obtient des cristaux jaunes de chlorhydrate (20,4 g) qui fondent avec décomposition à 152-154°C et dont l'analyse élémentaire calculée pour C₁₄H₂₄N₃O₄Cl est :

| | C% | H% | N% | O% | Cl% |
|---|---|---|---|---|---|
| Calculé : | 50,37 | 7,25 | 12,59 | 19,17 | 10,62 |
| Trouvé : | 50,60 | 7,19 | 12,48 | 19,35 | 10,68 |

### EXEMPLES D'APPLICATION

### EXEMPLE A

On prépare la composition tinctoriale suivante :
- Chlorhydrate de 1-(β-hydroxyéthyl)amino-4-(N-β-hydroxyéthyl, N-n-propyl)amino-2-nitrobenzène 0,96 g
- Monométhyléther de propylène glycol 10 g
- Diéthanolamide d'acides gras de Coprah 2 g
- Nonylphénol oxyéthyléné à 9 moles d'oxyde d'éthylène 8 g
- 2-amino 2-méthyl 1-propanol qs pH 9
- Eau qsp 100 g

On applique cette composition sur des cheveux gris naturels à 90% de blancs et on laisse poser 30 minutes à température ambiante. Après rinçage, les cheveux sont teints en cendré bleuté.

### EXEMPLE B

On prépare la composition tinctoriale suivante :
- Chlorhydrate de 1-(β-hydroxyéthyl)amino-4(N-β-hydroxyéthyl, N-n-butyl)amino-2-nitrobenzène 1 g
- Monométhyléther de propylèneglycol 10 g
- Diéthanolamide d'acides gras de Coprah 2 g
- Nonylphénol oxyéthyléné à 9 moles d'oxyde d'éthylène 8 g
- 2-amino-2-méthyl 1-propanol qs pH 9
- Eau qsp 100 g

Cette composition est appliquée sur des cheveux gris permanentés pendant 30 minutes à température ambiante. Après rinçage, les cheveux sont teints dans une couleur gris cendré violine.

### EXEMPLE C

On prépare la composition tinctoriale suivante :
- Chlorhydrate de 1-(β-hydroxyéthyl)amino-4-(N-β-hydroxyéthyl, N-isobutyl)amino-2-nitrobenzène 1 g
- Monométhyléther de propylène glycol 10 g
- Diéthanolamide d'acides gras de Coprah 2 g
- Nonylphénol oxyéthyléné à 9 moles d'oxyde d'éthylène 8 g
- 2-amino 2-méthyl 1-propanol qs pH 9
- Eau qsp 100 g

Cette composition est appliquée sur des cheveux gris permanentés pendant 30 minutes à température ambiante. Après rinçage, les cheveux sont teints en violine cendré.

### EXEMPLE D

On prépare la composition tinctoriale suivante:
- Chlorhydrate de 1-(β-hydroxyéthyl)amino-4(N-β-hydroxyéthyl, N-n-propyl)amino-2-nitrobenzène 0,3 g
- 1-β-hydroxyéthyloxy-3-méthylamino-4-nitrobenzène 0,08 g
- Nonylphénol oxyéthyléné à 9 moles d'oxyde d'éthylène 8 g
- Diéthanolamide d'acides gras de Coprah 2 g
- Ethylglycol 10 g
- Triéthanolamine qs pH 9
- Eau qsp 100 g

Cette composition est appliquée sur des cheveux gris naturels à 90% de blancs pendant 20 minutes à température ambiante. Après rinçage, les cheveux sont teints en blond clair mat doré.

## Revendications

1. 2-nitro p-phénylènediamine N¹,N⁴-dihydroxyéthylée ayant pour formule : 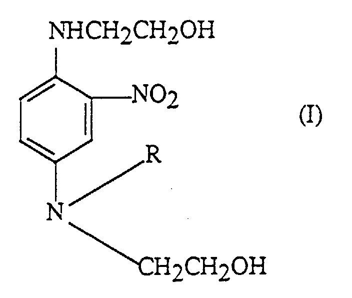 dans laquelle R représente un radical alkyle linéaire ou ramifié comportant 3 ou 4 atomes de carbone, et sels cosmétiquement acceptables de ce composé.

2. Composé selon la revendication 1, caractérisé par le fait que le radical alkyle R est choisi parmi les radicaux n-propyle, isopropyle, n-butyle, isobutyle et tert.-butyle.

3. Composé selon la revendication 1 ou 2, caractérisé par le fait qu'il est choisi parmi le 1-(β-hydroxyéthyl)amino-4-(N-β-hydroxyéthyl, N-n-propyl)amino-2-nitrobenzène, le 1-(β-hydroxyéthyl)amino-4-(N-β-hydroxyéthyl, N-n-butyl)amino-2-nitrobenzène et le 1-(β-hydroxyéthyl)amino-4-(N-β-hydroxyéthyl, N-isobutyl)amino-2-nitrobenzène et leurs sels cosmétiquement acceptables.

4. Composition tinctoriale pour la coloration directe de fibres kératiniques, et en particulier de cheveux humains, caractérisée par le fait qu'elle contient dans un véhicule aqueux, alcoolique ou hydroalcoolique, au moins un composé de formule (I) selon la revendication 1 ou l'un de ses sels cosmétiquement acceptables.

5. Composition tinctoriale selon la revendication 4, caractérisée par le fait qu'elle contient un composé de formule (I) choisi parmi le 1-(β-hydroxyéthyl)amino-4-(N-β-hydroxyéthyl, N-n-propyl)amino-2-nitro-benzène, le 1-(β-hydroxyéthyl)amino-4-(N-β-hydroxyéthyl, N-n-butyl)amino-2-nitrobenzène et le 1-(β-hydroxyéthyl)amino-4-(N-β-hydroxyéthyl, N-isobutyl)amino-2-nitrobenzène.

6. Composition tinctoriale selon la revendication 4 ou 5, caractérisée par le fait qu'elle contient au moins un composé de formule (I) ou l'un de ses sels cosmétiquement acceptables en association avec au moins un colorant nitré benzénique jaune ou jaune vert donnant une nuance selon Munsell comprise entre 2,5 Y et 2,5 GY sur cheveux gris à 90% de blancs.

7. Composition tinctoriale selon la revendication 6, caractérisée par le fait que le colorant nitré benzénique jaune ou jaune vert est choisi parmi les composés suivants: 1-β-hydroxyéthyloxy-3-méthylamino-4-nitrobenzène, 1-(méthylamino)-2-nitro-5-(β,γ-dihydroxypropyl)oxybenzène, 1-(β-hydroxyéthylamino)-2-méthoxy-4-nitrobenzène, 1-(β-aminoéthylamino)-2-nitro-5-méthoxy-benzène, 1,3-di(β-hydroxyéthylamino)-4-nitro-6-chloro-benzène, 1-amino-2-nitro-6-méthyl-benzène, 1-(β-hydroxyéthylamino)-2-hydroxy-4-nitro-benzène, N-(β-hydroxyéthyl)-2-nitro-4-trifluoro-méthyl-aniline, acide 4-β-hydroxyéthylamino-3-nitro-benzènesulfonique, acide 4-éthylamino-3-nitro-benzoïque, 4-(β-hydroxyéthyl)amino-3-nitro-chlorobenzène, 4-(β-hydroxyéthyl)amino-3-nitro-méthylbenzène, 4-(β,γ-dihydroxypropyl)amino-3-nitro-trifluorométhylbenzène, 1-β-uréidoéthylamino-4-nitro-benzène, O,N-bis(β-hydroxyéthyl)-2-amino-5-nitrophénol, 1,3-diamino-4-nitrobenzène, 1-hydroxy-2-amino-5-nitro-benzène, 1-amino-2-[tris(hydroxyméthyl)méthyl]amino-5-nitro-benzène, 1-(β-hydroxyéthyl)amino-2-nitrobenzène et 4-(β-hydroxy-éthylamino)-3-nitrobenzamide.

8. Composition tinctoriale selon l'une quelconque des revendications 4 à 7, caractérisée par le fait qu'elle contient en outre au moins un colorant nitré benzénique rouge choisi parmi les composés suivants : 1-hydroxy-3-nitro-4-(γ-hydroxypropylamino)benzène, N-(β-hydroxyéthyl)amino-3-nitro-4-aminobenzène, 1-amino-3-méthyl-4-(β-hydroxyéthyl)amino-6-nitro-benzène, 1-hydroxy-3-nitro-4-N-β-hydroxyéthyl aminobenzène, 1,4-diamino-2-nitrobenzène, 1-amino-2-nitro-4-méthylaminobenzène, N-(β-hydroxyéthyl)-2-nitro-paraphénylènediamine, 1-amino-2-nitro-4-(β-hydroxyéthylamino)-5-chlorobenzène, 2-nitro-4-amino-diphénylamine et 1-amino-3-nitro-6-hydroxybenzène.

9. Composition tinctoriale selon l'une quelconque des revendications 4 à 8, caractérisée par le fait qu'elle contient en outre au moins un colorant nitré benzénique orangé choisi parmi les composés suivants : 1-(β-aminoéthyl)amino-2-nitro-4-(β-hydroxyéthyl)oxybenzène, 1-(β,γ-dihydroxypropyl-oxy-3-nitro-4-(β-hydroxyéthyl)-amino-benzène, 1-hydroxy-3-nitro-4-aminobenzène, 1-hydroxy-2-amino-4,6-dinitrobenzène, 1-méthoxy-3-nitro-4-(β-hydroxyéthylamino)benzène, 2-nitro-4'-hydroxydiphénylamine et 1-amino-2-nitro-4-hydroxy-5-méthylbenzène.

10. Composition tinctoriale selon l'une quelconque des revendications 4 à 9, caractérisée par le fait qu'elle contient d'autres colorants directs choisis parmi les colorants azoïques, anthraquinoniques, les dérivés du triarylméthane et les colorants basiques.

11. Composition tinctoriale selon l'une quelconque des revendications 4 à 10, caractérisée par le fait qu'elle contient 0,01 à 10% en poids, et de préférence 0,1 à 5% en poids, exprimé en base libre, de composé de formule (I).

12. Composition tinctoriale selon l'une quelconque des revendications 4 à 11, caractérisée par le fait qu'elle contient 0,05 à 3% en poids de colorants nitrés benzéniques jaunes.

13. Composition tinctoriale selon l'une quelconque des revendications 4 à 12, caractérisée par le fait qu'elle contient 0,05 à 10% en poids d'autres colorants directs.

14. Composition tinctoriale selon l'une quelconque des revendications 4 à 13, caractérisée qu'elle contient des solvants organiques choisis parmi les alcools, glycols et éthers de glycols, en des concentrations comprises entre 0,5 et 20% en poids, et de préférence entre 2 et 10% en poids par rapport au poids total de la composition.

15. Composition tinctoriale selon l'une quelconque des revendications 4 à 14, caractérisée par le fait qu'elle contient au moins un adjuvant choisi parmi les amides gras en des concentrations comprises entre 0,05 et 10% en poids, les agents tensio-actifs anioniques, cationiques, non-ioniques, amphotères ou leurs mélanges, en des concentrations comprises entre 0,1 et 50% en poids, les épaississants en des concentrations comprises entre 0,2 et 5% en poids, les agents antioxydants, les parfums, les agents séquestrants, les agents filmogènes, les agents de traitement du cheveu, les agents dispersants, les agents de conditionnement du cheveu, les agents conservateurs et les agents opacifiants.

16. Composition tinctoriale selon l'une quelconque des revendications 4 à 15, caractérisée par le fait qu'elle a un pH compris entre 4 et 10.5, et de préférence entre 5 et 10.

17. Procédé de teinture des fibres kératiniques, et notamment des cheveux humains, par coloration directe, caractérisé par le fait qu'on applique la composition tinctoriale selon l'une quelconque des revendications 4 à 16 sur les fibres kératiniques sèches ou humides, et on sèche sans rinçage intermédiaire.

18. Procédé de teinture des fibres kératiniques, et notamment des cheveux humains par coloration directe, caractérisé par le fait qu'on applique la composition tinctoriale selon l'une quelconque des revendications 4 à 16 sur les fibres kératiniques sèches ou humides et qu'après avoir laissé agir la composition pendant 3 à 60 minutes, et de préférence pendant 5 à 45 minutes, on rince les fibres kératiniques, puis on les sèche.

## Patentansprüche

1. N¹,N⁴-Dihydroxyethyliertes 2-Nitro-p-phenylendiamin, das die Formel aufweist: 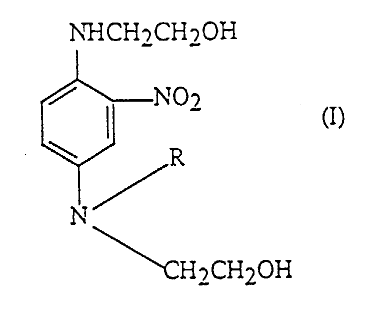 worin R einen linearen oder verzweigten Alkylrest mit 3 oder 4 Kohlenstoffatomen darstellt,
sowie kosmetisch geeignete Salze dieser Verbindung.

2. Verbindung gemäß Anspruch 1,
dadurch **gekennzeichnet,** daß
der Alkylrest aus n-Propyl, Isopropyl-, n-Butyl-, Isobutyl- und t-Butylresten ausgewählt ist.

3. Verbindung gemäß Anspruch 1 oder 2,
dadurch **gekennzeichnet,** daß
sie aus 1-(β-Hydroxyethyl)amino-4-(N-β-hydroxyethyl, N-n-propyl)amino-2-nitrobenzol, 1-(β-Hydroxyethyl)amino-4-(N-β-hydroxyethyl,N-n-butyl)amino-2-nitrobenzol und aus 1-(β-Hydroxyethyl)amino-4-(N-β-hydroxyethyl,N-isobutyl)amino-2-nitrobenzol sowie aus deren kosmetisch geeigneten Salzen ausgewählt ist.

4. Färbezusammensetzung zur Direktfärbung keratinischer Fasern und insbesondere der menschlichen Haare,
dadurch **gekennzeichnet**, daß
sie in einem wässrigen, alkoholischen oder hydroalkoholischen Trägermedium mindestens eine Verbindung der Formel (I) gemäß Anspruch 1 oder eines ihrer kosmetisch geeigneten Salze enthält.

5. Färbezusammensetzung gemäß Anspruch 4,
dadurch **gekennzeichnet,** daß
sie eine Verbindung der Formel (I) enthält, ausgewählt aus 1-(β-Hydroxyethyl)amino-4-(N-β-hydroxyethyl, N-n-propyl)amino-2-nitrobenzol, 1-(β-Hydroxyethyl)amino-4-(N-β-hydroxyethyl,N-n-butyl)amino-2-nitrobenzol und aus 1-(β-Hydroxyethyl)amino-4-(N-β-hydroxyethyl, N-isobutyl)amino-2-nitrobenzol.

6. Färbezusammensetzung gemäß Anspruch 4 oder 5,
dadurch **gekennzeichnet,** daß
sie mindestens eine Verbindung der Formel (I) oder eines ihrer kosmetisch geeigneten Salze in Abmischung mit mindestens einem nitrierten benzolischen gelben oder gelbgrünen Farbstoff enthält, was einen Farbton gemäß Munsell von 2,5 Y bis 2,5 GY auf grauen, zu 90% weißen Haaren ergibt.

7. Färbezusammensetzung gemäß Anspruch 6,
dadurch **gekennzeichnet**, daß
der nitrierte benzolische gelbe oder gelbgrüne Farbstoff aus den folgenden Verbindungen ausgewählt ist:
1-β-Hydroxyethyloxy-3-methylamino-4-nitrobenzol,
1- (Methylamino) -2-nitro-5- (β,γ-dihydroxipropyl) oxybenzol,
1-(β-Hydroxyethylamino)-2-methoxy-4-nitrobenzol,
1-(β-Aminoethylamino)-2-nitro-5-methoxybenzol,
1,3-Di(β-hydroxyethylamino)-4-nitro-6-chlorbenzol,
1-Amino-2-nitro-6-methylbenzol,
1-(β-Hydroxyethylamino)-2-hydroxy-4-nitrobenzol,
N-(β-Hydroxyethyl)-2-nitro-4-trifluormethylanilin,
4-β-Hydroxyethylamino-3-nitrobenzolsulfonsäure,
4-Ethylamino-3-nitrobenzoesäure,
4-(β-Hydroxyethyl)amino-3-nitrochlorbenzol,
4-(β-Hydroxyethyl)amino-3-nitromethylbenzol,
4-(β,γ-Dihydroxypropyl)amino-3-nitrotrifluormethylbenzol,
1-β-Ureidoethylamino-4-nitrobenzol,
O,N-Bis(β-hydroxyethyl)-2-amino-5-nitrophenol,
1,3-Diamino-4-nitrobenzol,
1-Hydroxy-2-amino-5-nitrobenzol,
1-Amino-2-(tris(hydroxymethyl)methyl)amino-5-nitrobenzol,
1-(β-Hydroxyethyl)amino-2-nitrobenzol und
4-(β-Hydroxyethylamino)-3-nitrobenzamid.

8. Zusammensetzung gemäß einem der Ansprüche 4 bis 7,
dadurch **gekennzeichnet,** daß
sie ausserdem mindestens einen nitrierten benzolischen roten Farbstoff enthält, ausgewählt aus den folgenden Verbindungen:
1-Hydroxy-3-nitro-4-(γ-hydroxypropylamino)benzol,
N-(β-Hydroxyethyl)amino-3-nitro-4-aminobenzol,
1-Amino-3-methyl-4-(β-hydroxyethyl)amino-6-nitrobenzol,
1-Hydroxy-3-nitro-4-N-β-hydroxyethylaminobenzol,
1,4-Diamino-2-nitrobenzol,
1-Amino-2-nitro-4-methylaminobenzol,
N-(β-Hydroxyethyl)-2-nitro-p-phenylendiamin,
1-Amino-2-nitro-4-(β-hydroxyethylamino)-5-chlorbenzol,
2-Nitro-4-aminodiphenylamin und
1-Amino-3-nitro-6-hydroxybenzol.

9. Färbezusammensetzung gemäß einem der Ansprüche 4 bis 8,
dadurch **gekennzeichnet,** daß
sie ausserdem einen nitrierten benzolischen orangenen Farbstoff enthält, der aus den folgenden Verbindungen ausgewählt ist:
1-(β-Aminoethyl)amino-2-nitro-4-(β-hydroxyethyl)oxibenzol,
1-(β,γ-Dihydroxypropyl)oxi-3-nitro-4-(β-hydroxyethyl)aminobenzol,
1-Hydroxy-3-nitro-4-aminobenzol,
1-Hydroxy-2-amino-4,6-dinitrobenzol,
1-Methoxy-3-nitro-4-(β-hydroxyethjyamino)benzol,
2-Nitro-4'-hydroxydiphenylamin und
1-Amino-2-nitro-4-hydroxy-5-methylbenzol.

10. Färbezusammensetzung gemäß einem der Ansprüche 4 bis 9,
dadurch **gekennzeichnet,** daß
sie weitere Direktfarbstoffe enthält, die aus Azo-Farbstoffen, Anthrachinon-Farbstoffen, Triarylmethanderivaten und aus basischen Farbstoffen ausgewählt sind.

11. Färbezusammensetzung gemäß einem der Ansprüche 4 bis 10,
dadurch **gekennzeichnet,** daß
sie 0,01 bis 10 und vorzugsweise 0,1 bis 5 Gew.% Verbindung der Formel (I), ausgedrückt als freie Base, enthält.

12. Färbezusammensetzung gemäß einem der Ansprüche 4 bis 11,
dadurch **gekennzeichnet,** daß
sie 0,05 bis 3 Gew.% nitrierte benzolische gelbe Farbstoffe enthält.

13. Färbezusammensetzung gemäß einem der Ansprüche 4 bis 12,
dadurch **gekennzeichnet,** daß
sie 0,05 bis 10 Gew.% weitere Direktfarbstoffe enthält.

14. Färbezusammensetzung gemäß einem der Ansprüche 4 bis 13,
dadurch **gekennzeichnet**, daß
sie organische Lösungsmittel, die aus Alkoholen, Glycolen und aus Glycolethern ausgewählt sind, in Konzentrationen von 0,5 bis 20 und vorzugsweise von 2 bis 10 Gew.% enthält, bezogen auf das Gesamtgewicht der Zusammensetzung.

15. Färbezusammensetzung gemäß einem der Ansprüche 4 bis 14,
dadurch **gekennzeichnet,** daß
sie mindestens einen Hilfsstoff enthält, der aus Fettamiden in Konzentrationen von 0,05 bis 10 Gew.%, aus anionischen, kationischen, nicht-ionischen und amphoteren oberflächenaktiven Mitteln oder deren Mischungen, in Konzentrationen von 0,1 bis 50 Gew.%, aus Verdickungsmitteln in Konzentrationen von 0,2 bis 5 Gew.%, aus Antioxidantien, Parfüm-Produkten, Sequestriermitteln, filmbildenden Mitteln, Haarbehandlungsmitteln, Dispergiermitteln, Haarkonditioniermitteln, Konservierungsmitteln und aus opak machenden Mitteln ausgewählt ist.

16. Färbezusammensetzung gemäß einem der Ansprüche 4 bis 15,
dadurch **gekennzeichnet**, daß
sie einen pH-Wert von 4 bis 10,5 und vorzugsweise von 5 bis 10 aufweist.

17. Verfahren zur Färbung keratinischer Fasern und insbesondere der menschlichen Haare durch Direktfärbung,
dadurch **gekennzeichnet,** daß
man die Färbezusammensetzung gemäß einem der Ansprüche 4 bis 16 auf die keratinischen trockenen oder feuchten Fasern aufbringt und sie ohne Zwischenspülung trocknen läßt.

18. Verfahren zur Färbung keratinischer Fasern und insbesondere der menschlichen Haare durch Direktfärbung,
dadurch **gekennzeichnet**, daß
man die Färbezusammensetzung gemäß einem der Ansprüche 4 bis 16 auf die keratinischen trockenen oder feuchten Fasern aufbringt und sie nach Einwirkung der Zusammensetzung über eine Dauer von 3 bis 60 und vorzugsweise von 5 bis 45 Minuten spült und dann trocknet.

## Claims

1. N¹,N⁴-Dihydroxyethylated 2-nitro-p-phenylenediamine having the formula: 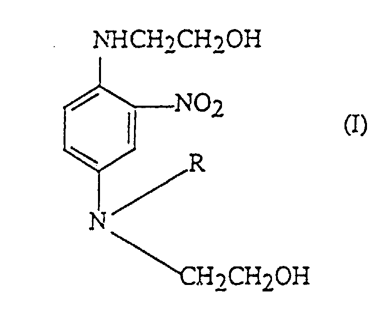 in which R represents a linear or branched alkyl radical containing 3 or 4 carbon atoms, and the cosmetically acceptable salts of this compound.

2. Compound according to Claim 1, characterized in that the alkyl radical R is chosen from the n-propyl, isopropyl, n-butyl, isobutyl and tert-butyl radicals.

3. Compound according to Claim 1 or 2, characterized in that it is chosen from 1-(β-hydroxyethyl)amino-4-(N-β-hydroxyethyl-N-n-propyl)amino-2-nitrobenzene, 1-(β-hydroxyethyl)amino-4-(N-β-hydroxyethyl-N-n-butyl)amino-2-nitrobenzeneandl-(β-hydroxyethyl)amino-4-(N-β-hydroxyethyl-N-isobutyl)amino-2-nitrobenzene and the cosmetically acceptable salts thereof.

4. Dye composition for the direct dyeing of keratin fibers, and in particular human hair, characterized in that it contains, in an aqueous, alcoholic or aqueous-alcoholic vehicle, at least one compound of formula (I) according to Claim 1, or one of the cosmetically acceptable salts thereof.

5. Dye composition according to Claim 4, characterized in that it contains a compound of formula (I) chosen from 1-(β-hydroxyethyl)amino-4-(N-β-hydroxyethyl-N-n-propyl) amino-2-nitrobenzene, 1-(β-hydroxyethyl) amino-4-(N-β-hydroxyethyl-N-n-butyl)amino-2-nitrobenzene and 1-(β-hydroxyethyl)amino-4-(N-β-hydroxyethyl-N-isobutyl)amino-2-nitrobenzene.

6. Dye composition according to Claim 4 or 5, characterized in that it contains at least one compound of formula (I), or one of the cosmetically acceptable salts thereof, in combination with at least one yellow or green-yellow nitrobenzene dye giving a Munsell shade of between 2.5 Y and 2.5 GY on gray hair containing 90% white hairs.

7. Dye composition according to Claim 6, characterized in that the yellow or green-yellow nitrobenzene dye is chosen from the following compounds: 1-β-hydroxyethyloxy-3-methylamino-4-nitrobenzene, 1-(methylamino)-2-nitro-5-(β,γ-dihydroxypropyl)oxybenzene, 1-(β-hydroxyethylamino)-2-methoxy-4-nitrobenzene, 1-(β-aminoethylamino) -2-nitro-5-methoxybenzene, 1,3-di(β-hydroxyethylamino)-4-nitro-6-chlorobenzene, 1-amino-2-nitro-6-methylbenzene, 1-(β-hydroxyethylamino)-2-hydroxy-4-nitrobenzene, N-(β-hydroxyethyl)-2-nitro-4-trifluoromethylaniline, 4-β-hydroxyethylamino-3-nitrobenzenesulfonic acid, 4-ethylamino-3-nitrobenzoic acid, 4-(β-hydroxyethyl)amino-3-nitrochlorobenzene, 4-(β-hydroxyethyl)-amino-3-nitromethylbenzene, 4-(β,γ-dihydroxypropyl)amino-3-nitrotrifluoromethylbenzene, 1-β-ureidoethylamino-4-nitrobenzene, O,N-bis(β-hydroxyethyl)-2-amino-5-nitrophenol, 1,3-diamino-4-nitrobenzene, 1-hydroxy-2-amino-5-nitrobenzene, l-amino-2-[tris(hydroxymethyl)methyl]-amino-5-nitrobenzene, 1-(β-hydroxyethyl)amino-2-nitrobenzene and 4-(β-hydroxyethylamino)-3-nitrobenzamide.

8. Dye composition according to any one of Claims 4 to 7, characterized in that it additionally contains at least one red nitrobenzene dye chosen from the following compounds: 1-hydroxy-3-nitro-4-(γ-hydroxypropylamino)-benzene, N- (β-hydroxyethyl)amino-3-nitro-4-aminobenzene, 1-amino-3-methyl-4-(β-hydroxyethyl)amino-6-nitrobenzene, 1-hydroxy-3-nitro-4-N-β-hydroxyethylaminobenzene, 1,4-diamino-2-nitrobenzene, 1-amino-2-nitro-4-methylaminobenzene, N- (β-hydroxyethyl)-2-nitro-para-phenylenediamine, l-amino-2-nitro-4-(β-hydroxyethylamino)-5-chlorobenzene, 2-nitro-4-amino-diphenylamine and 1-amino-3-nitro-6-hydroxybenzene.

9. Dye composition according to any one of Claims 4 to 8, characterized in that it additionally contains at least one orange nitrobenzene dye chosen from the following compounds: 1-(β-aminoethyl)amino-2-nitro-4-(β-hydroxyethyl)oxybenzene, 1-(β,γ-dihydroxypropyl-oxy-3-nitro-4-(β-hydroxyethyl)aminobenzene [sic], 1-hydroxy-3-nitro-4-aminobenzene, 1-hydroxy-2-amino-4,6-dinitrobenzene, 1-methoxy-3-nitro-4-(β-hydroxyethylamino)-benzene, 2-nitro-4'-hydroxydiphenylamine and 1-amino-2-nitro-4-hydroxy-5-methylbenzene.

10. Dye composition according to any one of Claims 4 to 9, characterized in that it contains other direct dyes chosen from azo dyes, anthraquinone dyes, triarylmethane derivatives and basic dyes.

11. Dye composition according to any one of Claims 4 to 10, characterized in that it contains 0.01 to 10% by weight, and preferably 0.1 to 5% by weight, expressed as free base, of a compound of formula (I).

12. Dye composition according to any one of Claims 4 to 11, characterized in that it contains 0.05 to 3% by weight of yellow nitrobenzene dyes.

13. Dye composition according to any one of Claims 4 to 12, characterized in that it contains 0.05 to 10% by weight of other direct dyes.

14. Dye composition according to any one of Claims 4 to 13, characterized in that it contains organic solvents chosen from alcohols, glycols and glycol ethers, in concentrations of between 0.5 and 20% by weight, and preferably of between 2 and 10% by weight, relative to the total weight of the composition.

15. Dye composition according to any one of Claims 4 to 14, characterized in that it contains at least one adjuvant chosen from fatty amides in concentrations of between 0.05 and 10% by weight, anionic, cationic, nonionic or amphoteric surface-active agents, or mixtures thereof, in concentrations of between 0.1 and 50% by weight, thickening agents in concentrations of between 0.2 and 5% by weight, antioxidants, fragrances, sequestering agents, film-forming agents, hair-treatment agents, dispersing agents, hair-conditioning agents, preserving agents and opacifying agents.

16. Dye composition according to any one of Claims 4 to 15, characterized in that it has a pH of between 4 and 10.5 and preferably between 5 and 10.

17. Process- for dyeing keratin fibers, and in particular human hair, by direct dyeing, characterized in that the dye composition according to any one of Claims 4 to 16 is applied to the dry or wet keratin fibers, and they are dried without intermediate rinsing.

18. Process for dyeing keratin fibers, and in particular human hair, by direct dyeing, characterized in that the dye composition according to any one of Claims 4 to 16 is applied to the dry or wet keratin fibers, and in that, after leaving the composition to act for 3 to 60 minutes, and preferably for 5 to 45 minutes, the keratin fibers are rinsed and then dried.
